# EUROPEAN PATENT APPLICATION

(11) **EP 4 710 936 A1**
(43) Date of publication of application: **18.03.2026**
(21) Application number: 24803524.8
(22) Date of filing: 09.05.2024
(51) Int. Cl.: A61K 39/145, A61K 39/215, A61P 31/14, A61P 31/16, A61P 37/04

(54) **COMBINATION VACCINE FOR PREVENTION OF INFLUENZA AND CORONAVIRUS INFECTIONS**

(30) Priority: 10.05.2023 JP 2023078218
(71) Applicant: National University Corporation Hokkaido University, Hokkaido 060-0808 (JP); KM Biologics Co., Ltd., Kumamoto-shi, Kumamoto 860-8568 (JP)
(72) Inventor: SHINGAI Masashi, Sapporo-shi Hokkaido 060-0808 (JP); KIDA Hiroshi, Sapporo-shi Hokkaido 060-0808 (JP); OGASAWARA Kazumasa, Sapporo-shi Hokkaido 060-0808 (JP); ENDO Masafumi, Kikuchi-shi, Kumamoto 869-1298 (JP); OKUMURA Minako, Kikuchi-shi, Kumamoto 869-1298 (JP); NAGASATO Toshiaki, Kumamoto-shi, Kumamoto 860-8568 (JP)
(74) Representative: Bandpay & Greuter
(86) International application number: PCT/JP2024/017278
(87) International publication number: WO 2024/232415

(57) **Abstract**

Disclosed is a combination vaccine for prevention of influenza and novel coronavirus infections. The combination vaccine for vaccination against influenza and novel corona viruses was obtained by inactivating all influenza virus particles and all novel coronavirus particles respectively, thereby inducing the antibodies to each virus without affecting each vaccine effect, so that the defensive effects were obtained against the attacks of each virus. In addition, the combination vaccine with this combination exhibited favorable neutralizing antibody induction and defensive effects to the attacks of these viruses even without addition of an adjuvant.

## Description

### [Technical Field]

The present invention relates to a combination vaccine for prevention of influenza and novel coronavirus infections.

### [Background Art]

Influenza viruses belongs to Orthomyxoviridae, which is classified as a ""enveloped, negative-sense, single-stranded RNA viruses" in the virus classification and are known to be classified into Types A, B, C, and D. Differences in these types are based on antigenic differences in the structural proteins M1 protein and nucleoprotein (NP) among the proteins constituting the virus particle. In addition to this, there are also pathological, morphological, and genetic differences, in particular, with significant differences between types A and B and types C and D. Types A and B are epidemics every year and are often the cause of influenza in humans.

The influenza virus was isolated from a pig in 1931, and human influenza virus was isolated in 1933. Around the same time, it was found that influenza viruses replicate in embryonated eggs, and this led to the use of inactivated whole particle virus vaccines, in which the virus was inoculated into embryonated eggs, cultured, harvested, purified, and then inactivated with formaldehyde. The inactivated whole particle virus vaccines are obtained by chemical inactivation of the purified virus particles with formaldehyde, so that the infectiousness is lost only by chemical modification of a virus gene or a virus protein thereby resulting in the vaccine antigen with keeping the particle form as it is. However, the inactivated whole particle virus vaccine in those early days had strong side effects such as an exothermic reaction, thereby leading to an idea to remove a lipid membrane component, which was believed to be the cause of it. The vaccine having a hemagglutinin (HA) of the influenza antigen as its main component, isolated by a surfactant after Zonal ultracentrifugal purification, and having the lipid membrane component which causes a fever removed by an ether, i.e., a prototype of the todays split vaccine, has a high safety. The split vaccine, which is a vaccine containing a purified HA (and NA) as its main component, has been produced since 1964 and the use thereof has been continued until today (NPL 1).

By the way, In recent years, inactivated whole-particle vaccines containing all virus particles have been considered to be highly immunogenic compared to the split vaccines consisting of some of the virus components, as they are able to induce a good immune response, particularly in naive individuals with no immunological memory, and therefore new researches and developments have been conducted on these vaccines (PTL 1, NPL 2). Of these, PTL 1 describes a precipitated inactivated influenza vaccine in which aluminum hydroxide gel is added to a whole particle influenza vaccine.

On the other hand, the novel coronavirus disease that emerged in 2019 has been officially named COVID-19 by the World Health Organization (WHO) and is caused by a virus called SARS-CoV-2. SARS-CoV-2 is a single-stranded positive-strand RNA virus with a diameter of 50 to 200 nm that belongs to the orthocoronavirus subfamily. Most COVID-19 infected individuals experience mild symptoms, such as no symptoms or cold-like symptoms, which resolve spontaneously, but severe cases may develop acute respiratory distress syndrome, sepsis, and/or multiple organ failure.

Vaccination is one method of preventing COVID-19. Various vaccines have been developed and approved for SARS-CoV-2, including, among others, an mRNA vaccine, a virus vector vaccine, and a gene-manipulated protein vaccine; and some of them have already been on the market (NPL 3). In Japan, too, the mRNA vaccine and the virus vector vaccine are administered to many people aged 5 and over, and it is hoped that the number of serious patient and death will be reduced by prevention of infection, deterioration, and onset. On the other hand, it is reported that these vaccines have side effects after the vaccination, such as pain at the vaccinated body part, headache, fatigue, muscle pain, anaphylaxis, thrombosis, pericarditis, and myocarditis. In addition, undiagnosable harmful actions have been known.

In addition to the vaccines above, an inactivated vaccine has been known. The inactivated vaccine is used, for example, as a seasonal influenza vaccine, a Japanese encephalitis vaccine, and a quadrivalent combination vaccine. The inactivated vaccines are produced by using a pathogen or a virus whose infectious power is removed, or using, among others, a component of the pathogen or part of the virus; thus, it is known that in general their side effects are low and the safety is high. Accordingly, it is expected that the inactivated SARS-CoV-2 vaccine may be used for infants younger than 5 years old as well. All the inactivated vaccines for SARS-CoV-2 are inactivated whole particle virus vaccines, and in overseas, VALNEVA (COVID-19 Vaccine (inactivated, adjuvanted) Valneva) and SINOVAC (Sinovac COVID-19 vaccine, CoronaVac/PiCoVacc) have already been approved in the production and sales thereof ahead of Japan (NPLs 4 and 5). To the VALNEVA vaccine are added aluminum hydroxide and CpG1018 (cytosine phospho-guanine) as the adjuvants, and to the SINOVAC vaccine is added aluminum hydroxide as the adjuvant. In Japan, KM Biologics is developing an inactivated (whole particle) COVID-19 vaccine called "KD-414" (currently undergoing Phase III trials). "KD-414" uses aluminum hydroxide as an adjuvant (NPL 6).

Before the pandemic declaration by the World Health Organization (WHO) in 2019, most of the respiratory infections were due to the seasonal influenza viruses including type A and type B influenzas. After the eruption of the COVID-19 pandemic, the incidence rate and mortality due to the seasonal influenza viruses dropped dramatically because of the restriction in the overseas travelling and social activities, among others. However, as a result of the release from these strict restriction in social activities, opening of overseas travelling, and recovery in economic activities, it is expected that diseases due to the seasonal influenza will increase. When considering the prediction of the continuing occurrence of the diseases due to COVID-19 in the future, both the diseases are significant threats to the public health; especially the risk of the severe infection may put a heavy load on the clinical practice. The SARS-CoV-2 and seasonal influenza have some common features such as multiplication pattern, sensitivity, clinical manifestations, and constant threat of novel variants/subtypes, so that a precautional strategy such as vaccination against the both viruses once every year may be necessary. Accordingly, WHO now recommends approved simultaneous administration of the vaccines of COVID-19 and seasonal influenza (NPL 7). There is a report indicating the immunogenicity and safety in the approved simultaneous dose of the vaccines of COVID-19 and seasonal influenza (NPL 8).

Given this background, hopes are high for a vaccine that can protect against both viruses with a single vaccination. Recently, it has been reported that an mRNA vaccine encoding the SARS-CoV-2 S protein and influenza hemagglutinin (HA) successfully protected against influenza and COVID-19 in a mouse model (NPL 9). However, as mentioned above, it has been pointed out that mRNA vaccines, particularly those with undiagnosable adverse effects, should be avoided annually.

The vaccine containing antigens to multiple infections (viruses), i.e., a combination vaccine, has a merit to provide the defensive capacity to multiple viruses with single vaccination, but on the other hand, there are problems to be solved as described below; thus, it is understood that the idea of simple combination alone cannot achieve this object. For example, it is difficult to predict immunological and biological actions of the vaccine, such as intervention among antigens and some other interactions, competition at the adjuvant adsorption site, and epitope-specific suppression; so, there is a case that the immunogenicity is decreased by the combination. Furthermore, single-antigen vaccine, which is developed on the presumption of the single vaccination, contains the optimized composition, especially the adjuvant; thus, there are concerns on undesirable interaction among these adjuvants as well as unexpected adjuvant effects.

### [Citation List]

### [Patent Literature]

PTL 1: WO 2008/041710A

### [Non Patent Literature]

NPL 1: YAKUGAKU ZASSHI, 131(12), 1723-1731 (2011) :
   https://www.jstage.jst.go.jp/article/yakushi/131/12/131_12_1723/_pdf/-char/ja
NPL 2: Vaccines 2022, 10(5), 804 https://www.mdpi.com/2076-393X/10/5/804
NPL 3: Clinical Immunology 222 (2021), 108634
   main.pdf (nih.gov)
NPL 4: Website of European Medicines Agency
   COVID-19 Vaccine (inactivated, adjuvanted) Valneva I European Medicines Agency (europa.eu)
NPL 5: Website of World Health Organization
   The Sinovac-CoronaVac COVID-19 vaccine: What you need to know (who.int)
NPL 6: Hum Vaccine Immunother. 2023 Apr 13;2193074
   Immunogenicity and safety of single booster dose of KD-414 inactivated COVID-19 vaccine in adults: An open-label, single-center, non-randomized, controlled study in Japan - PubMed (nih.gov)
NPL 7: SAGE Working Group on Influenza. Coadministration of seasonal inactivated influenza and COVID-19 vaccines: Interim guidance. World Health Organization
   Coadministration of seasonal inactivated influenza and COVID-19 vaccines (who.int)
NPL 8: Lancet Respir Med. 2022;10(2):167-79.
   Safety, immunogenicity, and efficacy of a COVID-19 vaccine (NVX-CoV2373) co-administered with seasonal influenza vaccines: an exploratory substudy of a randomised, observer-blinded, placebo-controlled, phase 3 trial (nih.gov)
NPL t 9: NPJ Vaccines. 2022 Jul 26;7(1):84.
   Rational development of a combined mRNA vaccine against COVID-19 and influenza - PMC (nih.gov)

### [Summary of the Invention]

The inventors of the present invention found that when a combination vaccine for vaccination against influenza and COVID-19 is obtained by inactivating both of them thereby making it to inactivated whole particle virus vaccine, it can induce neutralizing antibodies to the both viruses without losing the effects of the vaccine on the both viruses thereby enabling to obtain the defensive effects on the both viruses. In addition, it was confirmed that the combination vaccine with this combination can induce favorable neutralizing antibodies and express defensive effects to the attacks of these viruses even without the adjuvant addition. The present invention is based on these findings.

Accordingly, an object of the present invention is to provide a combination vaccine for prevention of the influenza and coronavirus infections.

The combination vaccine according to the present invention is characterized by that this contains an inactivated whole particle influenza vaccine and an inactivated whole particle SARS-CoV-2 vaccine.

According to the combination vaccine of the present invention, the influenza infection and the SARS-CoV-2 infection can be prevented by the single dose thereby realizing reduction in the vaccinated person's pain, time, and works, as well as the loads on doctors, among other things, due to the reduction in the number of the preventive vaccination.

### [Brief Description of the Drawings]

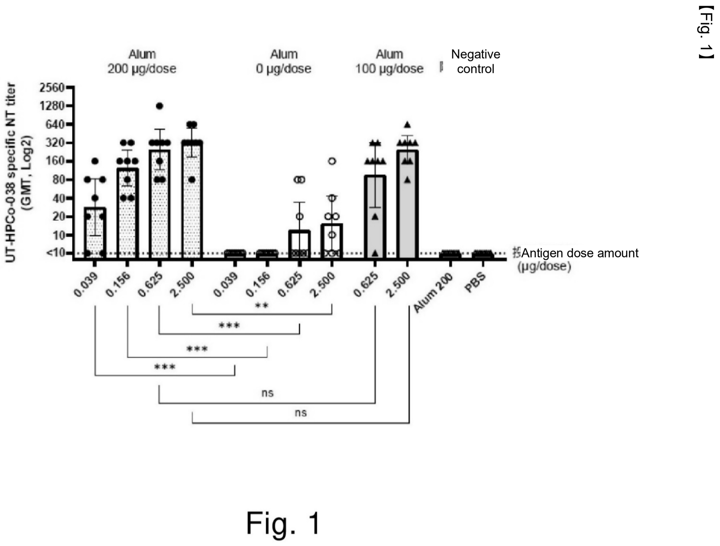

[Fig. 1] shows the result of a test to see whether the neutralizing antibody titers increase when various amounts of the inactivated whole particle SARS-CoV-2 vaccine and 0, 100 µg, or 200 µg of adjuvant were administered intraperitoneally to BALB/c mice (8 mice/group).

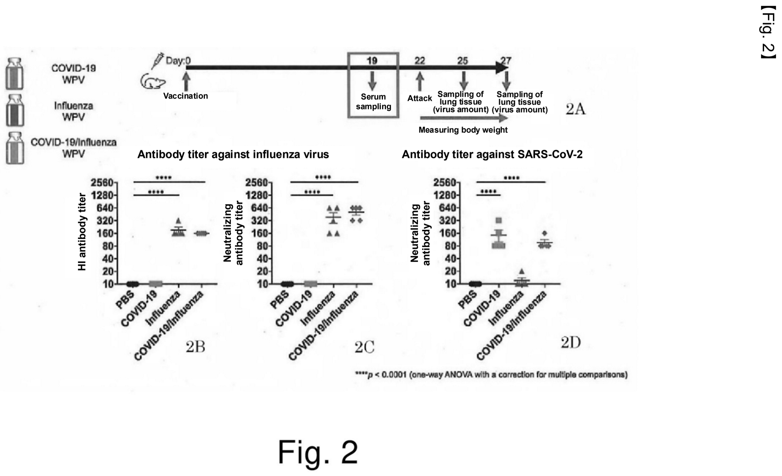

[Fig. 2] shows the detection of HI antibody titers or neutralizing antibody titers in the serum of 7-week-old female BALB/c mice 19 days after inoculation with the combination vaccine according to the present invention, i.e., the combination vaccine of the inactivated whole particle SARS-CoV-2 vaccine and the inactivated whole particle influenza vaccine (Figure 2A). The controls were the inactivated whole particle SARS-CoV-2 vaccine alone, the inactivated whole particle influenza vaccine alone, and PBS (Figures 2B to 2D).

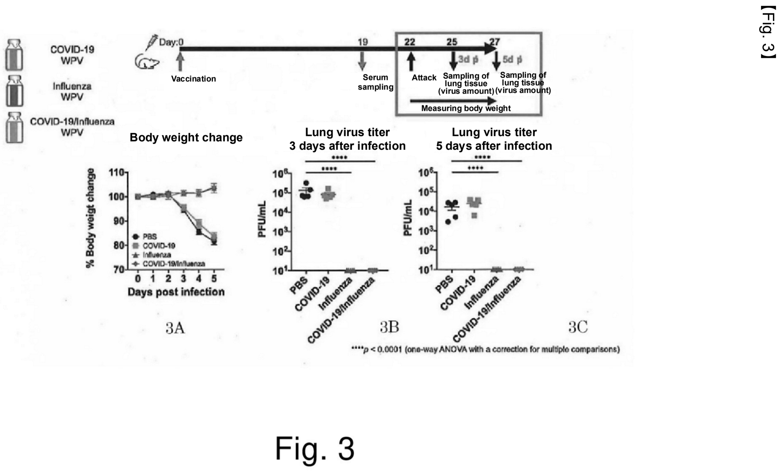

[Fig. 3] shows the results of a test, when the influenza virus was administered intranasally to the BALB/c mice that had been vaccinated with the combination vaccine of the present invention, the inactivated whole particle SARS-CoV-2 vaccine, and the inactivated whole particle influenza vaccine individually, and PBS, to measure the body weight change (Figure 3A) and the lung virus titers at 3 days and 5 days after the infection (dpi) (Figures 3B and C).

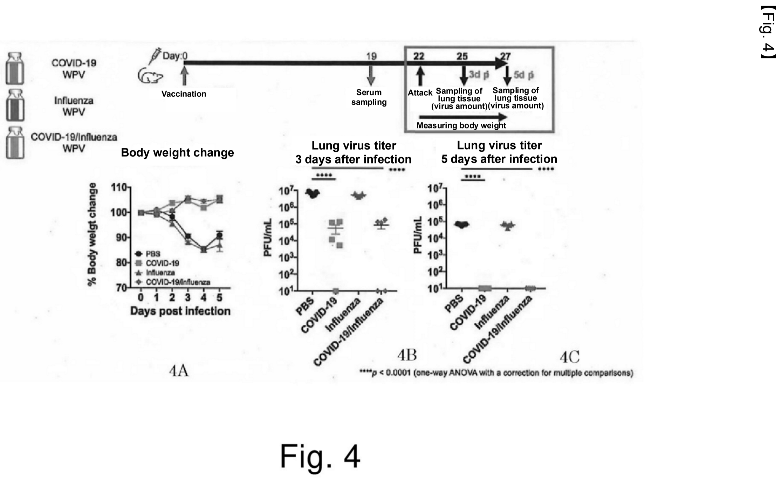

[Fig. 4] shows the results of a test, when the SARS-CoV-2 Wuhan strain was administered intranasally to the BALB/c mice that had been administered with the combination vaccine of the present invention, the inactivated whole particle SARS-CoV-2 vaccine, and the inactivated whole particle influenza vaccine individually and PBS, to measure the body weight change (Figure 4A) and the lung virus titers at 3 days and 5 days after the infection (dpi) (Figures 4B and C).

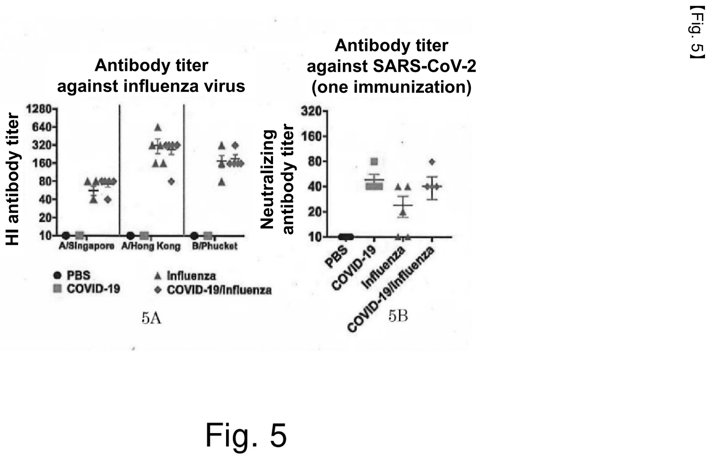

[Fig. 5] shows the HI antibody titer or neutralizing antibody titer in the serum of 7-week-old female BALB/c mice 22 days after they were inoculated with a combination vaccine of inactivated whole particle SARS-CoV-2 vaccine and tetravalent inactivated whole particle influenza vaccine strains (A/Singapore, A/Hong Kong, B/Phuket, B/Texas) as the combination vaccine according to the present invention.

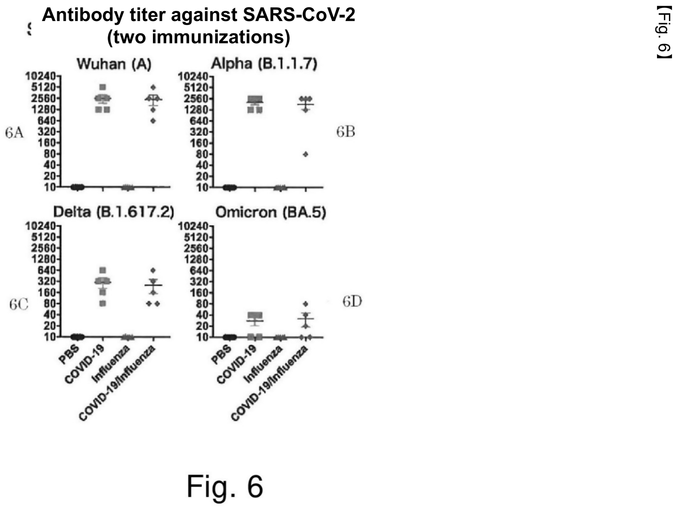

[Fig. 6] shows the results of evaluating the neutralizing antibodies against the alpha strain, the delta strain, and the omicron strain, which are variants of the Wuhan vaccine strain, in the serum sample obtained from the mouse at 36 days after administration of a combination vaccine of inactivated whole particle SARS-CoV-2 vaccine and tetravalent inactivated whole particle influenza vaccine, as the combination vaccine according to the present invention.

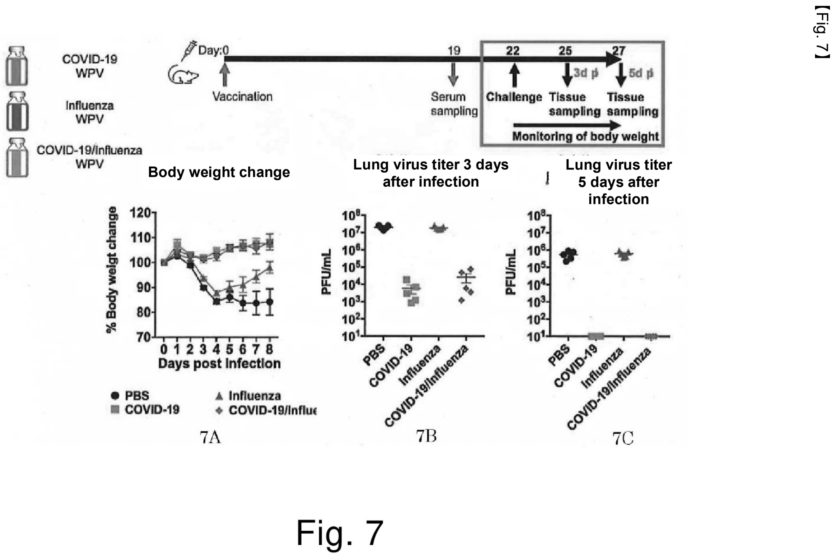

[Fig. 7] shows the results of a test, when the SARS-CoV-2 Wuhan strain was administered intranasally to the BALB/c mice that had been inoculated with a combination of inactivated whole particle SARS-CoV-2 vaccine and the quadrivalent inactivated whole particle influenza vaccine as the combination vaccine of the present invention, and the inactivated whole particle SARS-CoV-2 vaccine and the quadrivalent inactivated whole particle influenza vaccine individually, and PBS, to measure the body weight change (Figure 7A) and the lung virus titers at 3 days and 5 days after the infection (dpi) (Figures 7B and C).

### [Description of the Invention]

### Definition

In this specification, the term "influenza virus" is used with the same meaning as "seasonal influenza virus" unless otherwise specifically noted.

In this specification, the term "SARS-CoV-2" is used with the same meaning as "novel coronavirus" unless otherwise specifically noted.

### Combination vaccine according to the present invention

The combination vaccine according to the present invention contains basically an inactivated whole particle influenza vaccine and an inactivated whole particle SARS-CoV-2 vaccine, and is further characterized by not containing an adjuvant.

As described above, it was understood that a combination vaccine could not be realized by simply combining viruses, but by using inactivated whole particle vaccines for both the influenza vaccine and the SARS-CoV-2 vaccine, a combination vaccine was obtained that induces neutralizing antibodies against each virus without affecting the efficacy of each vaccine, and furthermore, provides protection against attack by each virus. This fact should be acknowledged as an unexpected fact to those skilled in the art.

Furthermore, the combination vaccine according to the present invention does not contain an adjuvant. The combination vaccine according to the present invention "does not contain an adjuvant" means that a substance that enhances a humoral immunity and a cellular immunity is substantially not included therein due to co-administration of the antigen. Here, the term "substantially" means that unavoidable amount thereof may be allowed, but effective amount to enhance the humoral immunity and cellular immunity is not included.

As described above, the inactivated whole particle virus vaccines for SARS-CoV-2 that have been reported, developed, or produced and sold until to date contain an adjuvant such as aluminum hydroxide, which is the result of the determination that adjuvants are necessary for immunogenicity. While it was previously understood that inactivated whole particle vaccines against SARS-CoV-2 require adjuvants, the combination vaccine of the present invention, when combined with an inactivated whole virus influenza vaccine, does not require the addition of an adjuvant and does not affect the efficacy of each vaccine, thereby inducing neutralizing antibodies against each virus and providing protection against attack by each virus. This fact, too, should be acknowledged as an unexpected fact to those skilled in the art.

The combination vaccine according to the present invention can prevent influenza infection and COVID-19 with a single vaccination, reducing the number of vaccinations required and reducing pain, time, labor, etc. for the vaccine recipient. It can also reduce the burden on doctors and others.

Furthermore, compared to split vaccines consisting of some of the virus components, inactivated whole virus vaccines containing all virus particles are thought to be able to induce a good immune response, particularly in naive individuals who have no immunological memory, and the combination vaccine using the inactivated whole virus vaccine of the present invention is also thought to be advantageous in terms of high immunogenicity.

### Inactivated whole particle influenza vaccine

The "inactivated whole particle influenza vaccine" that is included in the combination vaccine according to the present invention means the influenza virus having been inactivated while retaining the form thereof.

The term "influenza virus" in this specification means all the viruses having been known until today, including the type A influenza, the type B influenza, the type C influenza, the type D influenza, and all the subtypes of these viruses, as well as those that may appear and be classified as the influenza virus in the future.

In the present invention, the term "inactivated whole particle influenza vaccine" may include not only a single whole particle influenza virus which is inactivated, but also include inactivated multiple whole particle influenza viruses.

According to a preferred embodiment of the present invention, the inactivated whole particle influenza vaccine is the inactivated whole particle virus of at least any one of the type A influenza virus and the type B influenza virus; preferably the both. Two subtypes of the type A influenza virus or of the type B influenza virus, or even more than these may be included. For example, a quadrivalent inactivated whole particle influenza vaccine containing two type A virus strains and two type B virus strains can be used as the inactivated whole particle influenza vaccine in the combination vaccine according to the present invention.

The "inactivated whole particle influenza vaccine" included in the combination vaccine according to the present invention may be produced by known methods, for example, in accordance with the method described in Vaccine, 2022, 10(5), 804 (NPL 2).

Specifically, the influenza virus is cultured. In the case of multiple influenza viruses, it is preferable to culture them individually. The culturing may be performed by known "embryonated egg method" or "cell culture method"; in general, the "embryonated egg method" is used. Next, the ulantoic fluid containing the multiplied viruses is purified and concentrated by the method such as a sucrose density gradient centrifugation method to obtain the virus particles.

Next, the resulting virus particles are inactivated. The inactivation can be performed by contacting an inactivating agent with the virus solution containing the virus particles. E examples of the inactivating agent include formaldehyde, paraformaldehyde, glutaraldehyde, dicyclohexyl carbodiimide (DCC), diisopropyl carbodiimide (DIC), 1-ethyl-3-(3-dimethlaminopropyl) carbodiimide hydrochloride, and β-propiolactone. The inactivating agent is preferably formaldehyde.

Then, if necessary, the solution is diluted with a phosphate buffer solution containing sodium chloride such that a prescribed amount of the inactivated whole influenza virus will be included therein.

### Inactivated whole particle SARS-CoV-2 vaccine

The term "inactivated whole particle SARS-CoV-2 vaccine" that is included in the combination vaccine according to the present invention means the SARS-CoV-2 virus particles which have been inactivated while retaining the form thereof.

The term "SARS-CoV-2" in this specification means all the SARS-CoV-2 strains having been known until today, including, for example, the Wuhan strain and its variants, i.e., an alpha strain, a beta strain, a gamma strain, a delta strain, and an omicron strain, as well as those that may appear and be classified as the SARS-CoV-2 in the future.

In the present invention, the "inactivated whole particle SARS-CoV-2 vaccine" may include not only a single inactivated whole virus SARS-CoV-2 virus but also multiple inactivated whole virus SARS-CoV-2 viruses.

The "inactivated whole particle SARS-CoV-2 vaccine" included in the combination vaccine according to the present invention can be produced by known methods, for example, by the method described in Hum Vaccine Immunother. 2023 Apr 13; 2193074 (NPL 6).

Specifically, after the SARS-CoV-2 is cultured by the "cell culturing method", that is, after the virus strain is inoculated in a culture cell and then cultured, the virus suspension is clarified, concentrated, purified, and inactivated to produce the virus solution containing the virus particles. Cells to be used for culture include cells that allow good viral growth and produce highly immunogenic antigens, and for example, cells derived from animals other than humans (e.g., chickens) or animal-derived cell lines (animal cell lines) can be used. Specific examples of the cell include Calu-3 cell and A549 cell, which are derived from a human lung epithelial adenocarcinoma cell, and Vero cell, Vero E6 cell, and Vero E6/TMPRSS 2 cell, which are derived from an African green monkey kidney. Vero cell may be preferrable. Furthermore, purification of virus particles can be carried out using means such as ultracentrifugation such as sucrose density gradient centrifugation, ultrafiltration or filtration, and liquid chromatography.

The resulting virus particles are then inactivated. The inactivation may be carried out in substantially the same manner as the inactivation of the influenza virus as described above, i.e., by contacting a virus solution containing the virus particles with the inactivating agent. Examples of the inactivating agent include formaldehyde, paraformaldehyde, glutaraldehyde, dicyclohexyl carbodiimide (DCC), diisopropyl carbodiimide (DIC), 1-ethyl-3-(3-dimethlaminopropyl) carbodiimide hydrochloride, and β-propiolactone. The inactivating agent is preferably formaldehyde.

### Combination vaccine formulation and Use thereof

The combination vaccine according to the present invention comprises a combination of the inactivated whole particle influenza vaccine and the inactivated whole particle SARS-CoV-2 vaccine. In addition to the vaccine formulation containing a mixture of the both vaccines, the combination may include the embodiment in which these vaccines are prepared by filling each vaccine separately into respective vials, and at the time of the use thereof, they are mixed to make the combination vaccine. According to a preferred embodiment of the present invention, the both vaccines are mixed so as to provide the vaccines as a single vaccine formulation.

The combination vaccine of the present invention does not require an adjuvant, but may contain components other than an adjuvant. For example, it may contain a carrier acceptable for vaccine formulations, specifically saline, buffered saline, sugars such as dextrose, amino acids, water, glycerol, isotonic aqueous buffers, surfactants, and combinations thereof. It may also contain preservatives (e.g., thimerosal), isotonicity agents, pH adjusters, and inactivating agents (e.g., formalin).

In the production of the combination vaccine according to the present invention, the purified inactivated whole virus vaccines may be mixed with other component after replacing, as needed, with an appropriate buffer by ultrafiltration or the like followed by a sterile filtration by means of a membrane filter, or mixed with other component followed by a sterile filtration by means of a membrane filter.

The combination vaccine according to the present invention may be administered systemically or topically. The topical administration includes intramuscular, subcutaneous, intradermal, intranasal, and pharyngeal administration, and can be administered by injection, spray, or application.

The concentration of the inactivated whole particle influenza vaccine in the combination vaccine according to the present invention is in general preferably about 0.3 to about 3.0 µg/mL, more preferably about 0.7 to about 2.0 µg/mL. The dose of the inactivated whole particle influenza vaccine according to the combination vaccine of the present invention is determined as appropriate with considering the age, gender, body weight, and the like of the object; usually, the dose as the antigen is about 3 to about 15 µg, preferably about 7 to about 11 µg. When the combination vaccine according to the present invention contains multiple virus strains in the inactivated whole particle influenza vaccine, the same range as above per one virus strain may be applied.

The concentration of the inactivated whole particle SARS-CoV-2 vaccine in the combination vaccine according to the present invention is in general preferably about 0.3 to about 3.0 µg/mL, more preferably about 0.7 to about 2.0 µg/mL. The dose of the inactivated whole particle SARS-CoV-2 vaccine according to the combination vaccine of the present invention is determined as appropriate with considering the age, gender, body weight, and the like of the object; usually, the dose as the antigen is about 3 to about 15 µg, preferably about 7 to about 11 µg.

The combination vaccine according to the present invention may also be administered two or more times with intervals between the vaccinations.

### [Examples]

The present invention will be described in detail by means of Examples, but the present invention is not limited at all to these Examples.

In the description of Examples below, following abbreviations are used:
- Inactivated whole particle influenza vaccine: Flu-WPV
- Quadrivalent inactivated whole particle influenza vaccine: qFlu-WPV
- Inactivated whole particle SARS-CoV-2 vaccine: Co-WPV
- Combination vaccine of inactivated whole particle SARS-CoV-2 vaccine and inactivated whole particle influenza vaccine (bivalent vaccine): Co/Flu-WPV
- Combination vaccine of inactivated whole particle SARS-CoV-2 vaccine and quadrivalent inactivated whole particle influenza vaccine: Co/qFlu-WPV

### Reference Example 1: Necessity of aluminum hydroxide adjuvant in the inactivated whole particle SARS-CoV-2 vaccine

The inactivated whole particle SARS-CoV-2 vaccine was prepared from the SARS-CoV-2 virus strain according to the description in Hum Vaccine Immunother. 2023 Apr 13; 2193074 (NPL 6). In paricular, the Vero cell was cultured in the Vero cell culture medium containing microcarriers; then, the virus solution was harvested from the resulting virus suspension. After concentration, it was inactivated to obtain the inactivated SARS-CoV-2 antigen API (inactivated whole novel coronavirus vaccine).

A 500 µL solution containing the immune substance (antigen API and aluminum hydroxide adjuvant) with the amount described in Table 1 was administered intraperitoneally to BALB/c mice (8 mice/group) twice with the interval of 2 weeks; then, the blood was collected 2 weeks after the second administration. A serum was centrifugally separated from the obtained blood, and then this was cryopreserved. This serum was used for the neutralizing antibody titer test. From the neutralizing antibody titer obtained, the geometric mean value and the 95% confidence interval of the neutralizing antibody titers in each dosed group were calculated using the Excel's GEOMEAN function and CONFIDENCE. T function. Using the medical statistics analysis software "GRAPHPAD Prism 9 (ver. 9. 3.1)", the graph of the geometric mean value of the neutralizing antibody titer of each dose group was obtained, and then, the significance difference test (Unpaired Mann-Whitney test) among the same antigen dose was conducted (**p value 0.001 to 0.01, ***p <0.0001). The results are described in Figure 1. From this, the tendency was recognized that with the increase in the dose of the antigen, the neutralizing antibody titer increases. The significance difference test also indicated the significant difference in all the dose groups. Because the neutralizing antibody titer increased significantly by the adjuvant addition, it was judged that the adjuvant is necessary in the inactivated whole novel coronavirus vaccine.

**[Table 1]**

| | | | | | | | | | | | | |
|---|---|---|---|---|---|---|---|---|---|---|---|---|
| Antigen (µg/dose) | 0.039 | 0.156 | 0.625 | 2.5 | 0.039 | 0.156 | 0.625 | 2.5 | 0.625 | 2.5 | 0 | 0 PBS |
| Adjuvant (µg/dose) | 200 | 200 | 200 | 200 | 0 | 0 | 0 | 0 | 100 | 100 | 200 | 0 |

### Example 1: Preparation of inactivated whole particle SARS-CoV-2 vaccine (Co-WPV) and inactivated whole particle influenza vaccines (Flu-WPV and qFlu-WPV)

Co-WPV was prepared as follows. The SARS-CoV-2 Wuhan virus strain was inoculated to the Vero cell to multiply the virus; and after culturing at 37°C for 2 days, it was centrifugated at 4°C and 3000 rpm for 30 minutes. Next, to the resulting supernatant was added aqueous 10% formaldehyde solution (final concentration of 0.1%), and then this was incubated at 4°C for 1 week to inactivate the virus. After the inactivation, the virus was concentrated by centrifugation at 4°C and 19,000 rpm for 1.5 hours, and then, purified by the sucrose density ultracentrifugation at 4°C and 27,000 rpm for 1.5 hours. The total protein content was measured by a spectrophotometer.

Flu-WPV (A/California 9 µg) was prepared from A/California in accordance with the description, Vaccine, 2022, 10(5), 804 (NPL 2).

The quadrivalent inactivated whole particle influenza vaccine (qFlu-WPV: 9 µg for each of the four influenza vaccine strains) containing the 2017-2018 virus strains (A/Singapore, A/Hong Kong, B/Phuket, and B/Texas) (NPL 2) was prepared.

Co-WPV, Flu-WPV, and qFlu-WPV each were stored at 4°C in the vial for each.

### Example 2: Preparation of combination vaccine of inactivated whole particle SARS-CoV-2 vaccine and inactivated whole particle influenza vaccine (bivalent vaccine: Co/Flu-WPV), and HI antibody titer or serum antibody titer

Co-WPV and Flu-WPV described in Example 1 were mixed to prepare Co/Flu-WPV (Co-WPV: 9 µg, Flu-WPV (A/California): 9 µg). The antigens with the same amount were used in order to induce the antibodies against each virus in the same way. The adjuvant was not added.

As the controls, Co-WPV (SARS-CoV-2: 9 µg), Flu-WPV (A/California: 9 µg), and PBS each were administered to 7-weeks old BALB/c female mice (Figure 2A). A serum was collected from the caudal vena cava at 19 days after the vaccination, and then the HI antibody titers or the neutralization antibody titers were measured. The results are described in Figures 2B to D. The dots indicate the individual values and the line indicates the SEM average. For the statistical analysis, One-way ANOVA by the Turkey's multiple comparison test was used (**** p <0.0001).

The mice inoculated with Flu-WPV or Co-WPV induced significantly high level of the HA inhibition (HI) and the neutralizing antibody against A/California as compared with the mice inoculated with PBS (Figures 2B and C). The neutralizing antibody titers in the mice inoculated with Flu-WPV or Co/Flu-WPV were in the range of 160 to 640, and statistically there was no significant difference among all the mice inoculated with them. In the animals inoculated with any one of PBS or Co-WPV, the HI antibody or the neutralizing antibody against the influenza virus was not detected.

The neutralizing antibody titers against SARS-CoV-2 in the animals inoculated only with Co-WPV were in the range of 80 to 320, and in the animals inoculated with Co/Flu-WPV, the iters were in the range of 80 to 160; so, these were significantly higher than the animals inoculated with PBS (Figure 2D). From these results, it became clear that the bivalent vaccine of Flu-WPV and Co-WPV does not exhibit clear intervention effect on the antibody induction against each antigen, and that the antibodies can be sufficiently induced even without the adjuvant addition.

### Example 3: Attacking tests by influenza virus and SARS-CoV-2

To the BALB/c mice having been administered with each vaccine and PBS in Example 2, the influenza A/California virus with 3000 PFU or the SARS-CoV-2 Wuhan strain with 10⁵ PFU was intranasal administered at 22 days after the vaccination; then, the body weight change of each was monitored every day. Also, the lung virus titer was measured at 3 days and 5 days after the infection (dpi). Specifically, some mice were mercifully killed with an animal-welfare oriented way at 3 days after the virus attack, and the lung tissue was taken out. The left lung was put into formalin in order to make a tissue section. On the other hand, the right lung was divided into two pieces in order to measure the virus titer and the gene expression of infectious cytokines and chemokines. The influenza virus amount in the lung was measured by a usual method. The attacking test results of the influenza virus are described in Figures 3A to C, and the attacking test results of SARS-CoV-2 are in Figures 4A to C. The dots indicate the individual values and the line indicates the SEM average. For the statistical analysis, One-way ANOVA by the Turkey's multiple comparison test was used (**** p <0.0001).

As the results of the attacking test of the influenza virus, the body weight of the mice inoculated with PBS and Co-WPV showed dramatic reduction of 20% from the initial measurement value before the virus dosing, but the body weight of the mice inoculated with the Flu-WPV and Co/Flu-WPV changed within a normal range (Figure 3A). In order to evaluate the effectiveness of Co/Flu-WPV in suppression of the virus multiplication, the virus titter in the lung was measured at 3 days and 5 days after the infection (dpi). The virus titers in the lung homogenate in the PBS group and the Co-WPV group were both about 10⁵ PFU/ml at 3 dpi and about 10⁴ PFU/ml at 5 dpi (Figures 3B and C). On the contrary, in the mouse lung inoculated with Flu-WPV or Co/Flu-WPV, the virus titers were below the detection limit (Figures 3B and C). From these results, it was indicated that the virus multiplication was effectively suppressed in the mouse lung inoculated with Flu-WPV or Co/Flu-WPV.

From the results of the attacking test of SARS-CoV-2, the animals inoculated with Flu-WPV or PBS lost their body weights of about 15% from the initially measured value before the virus dosing; but the animals immunized with Co-WPV or Co/Flu-WPV did not reduce their body weights over the observation period (Figure 4A). At 3 days and 5 days after the infection (dpi), some animals in each group were mercifully killed with an animal-welfare oriented way, and the lung virus titers were measured using the Plaque assay. At 3 dpi, in the animals inoculated with Co-WPV or Co/Flu-WPV, the virus titers were about 100 times lower than the animals inoculated with PBS or Flu-WPV (Figure 4B). Furthermore, at 5 dpi, the virus titer of about 10⁵ PFU/ml was detected in the group inoculated with PBS or Flu-WPV, but the virus titers were below the detection limit in the Co-WPV group and the Co/Flu-WPV group (Figures 4B and C).

### Example 4: Preparation of combination vaccine of inactivated whole particle SARS-CoV-2 vaccine and quadrivalent inactivated whole particle influenza vaccine (Co/qFlu-WPV), and HI antibody titer or serum antibody titer

Co/qFlu-WPV was prepared by mixing Co-WPV (SARS-CoV-2: 9 µg) and qFlu-WPV (four influenza vaccine strains: 9 µg each) described in Example 1.

Co/qFlu-WPV, and as the controls, Co-WPV (SARS-CoV-2: 9 µg), qFlu-WPV (9 µg each), and PBS were inoculated to 7-weeks old BALB/c female mice. A serum was collected from the caudal vena cava at 22 days after the dosing, and then the HI antibody titers or the neutralization antibody titers were measured. The results are described in Figure 5. The dots indicate the individual values and the line indicates the SEM average. For the statistical analysis, One-way ANOVA by the Turkey's multiple comparison test was used (**** p <0.0001).

The mice inoculated with qFlu-WPV or Co/ qFlu-WPV all induced the neutralizing antibody against each virus antigen, and there were no statistical difference recognized among the mice inoculated with only qFlu-WPV or with Co/ qFlu-WPV (Figure 5A).

In the mice inoculated with Co-WPV or Co/qFlu-WPV, production of the neutralizing antibody against SARS-CoV-2 was recognized in the range of 40 to 80 in the both groups, but the neutralizing antibody titers in the mice of the PBS group were below the detection limit (Figure 5B). These results indicate that Co/qFlu-WPV induces the humoral immunity against each virus without clear intervention among these inactivated whole particle virus vaccines.

In order to evaluate the neutralizing antibody against the SARS-CoV-2 variants, i.e., the Wuhan strain (A), the alpha strain (B.1.1.7), the delta strain (B.1.6.17.2), and the omicron strain (BA. 5), the above-mentioned mice were additionally immunized at the 22^{nd} day; and at 36 days thereafter, serum samples were collected from each mouse. The results are described in Figure 6. The dots indicate the individual values and the line indicates the SEM average. For the statistical analysis, One-way ANOVA by the Turkey's multiple comparison test was used (**** p <0.0001).

As a result of the neutralization test after the second vaccination, it was recognized that the mice inoculated with Co-WPV and Co/qFlu-WPV induced the antibodies against the Wuhan strain and the alpha strain. The titers to the Wuhan strain reached 2560 and 2463, respectively in average, and 2048 and 1808, respectively, for the alpha strain (Figures 6A and B). Notably, in the mice inoculated with Co-WPV or Co/qFlu-WPV, statistically there was no significant difference recognized between the antibodies against the Wuhan strain and the alpha strain. In the mice inoculated with Co-WPV or Co/qFlu-WPV, high neutralizing antibodies against the delta strain were also detected with the average of 304 and 256, respectively (Figure 6C). On the other hand, the neutralizing antibodies of the mice inoculated with PBS or qFlu-WPV were below the detection limit in all the mice tested. These results suggest that Co/qFlu-WPV can effectively induce the cross-reactive neutralizing antibody.

Also, the neutralizing antibodies against the omicron strain were measured (Figure 6D). Contrary to the strong antibody induction against the alpha strain and the delta strain in the mice inoculated with Co-WPV and Co/qFlu-WPV, the antibodies against the omicron strain was low; in some mice, the level thereof was below the detection limit. From this, the possibility was suggested that the two-time dose method is not sufficient to enhance the immunity against the omicron strain.

### Example 5: Attacking test of SARS-CoV-2 in Co/qFlu-WPV

Similarly to Example 3, the defensive effect of the Co/qFlu-WPV against the attack of the SARS-CoV-2 infection was studied. The results are described in Figure 7. The dots indicate the individual values and the line indicates the SEM average. For the statistical analysis, One-way ANOVA by the Turkey's multiple comparison test was used (**** p <0.0001).

After the mice were Intranasal administered with 10⁵ PFU SARS-CoV-2, all the mice of the control PBS and inoculated with Flu-WPV decreased their body weights rapidly until 4 dpi (Figure 7A). On the other hand, in the mice inoculated with Co-WPV or Co/qFlu-WPV, the decrease in the body weight was not recognized after the virus was inoculated (Figure 7A).

From the measurement using the Plaque assay, in the mice inoculated with PBS and qFlu-WPV, the lung virus titers of 2x10⁷ and 1.9x10⁷ PFU, respectively, in average were recognized at 3 dpi, which were 100 times higher than the virus titers of the mice inoculated with Co-WPV or Co/qFlu-WPV. At 5 dpi, in the mice belonging to the PBS group and the qFlu-WPV group, the titers were 5.5x10⁵ and 6.2x10⁵ PFU, respectively, in average; but in the mice inoculated with Co-WPV or Co/qFlu-WPV, the virus titers were not recognized (Figures 7B and C).

As can be seen above, the combination vaccine according to the present invention induces the neutralizing antibodies to each virus without affecting each vaccine effect thereby showing the defensive effects to the attack of each virus. Furthermore, the addition of the adjuvant was not necessary. With regard to SARS-CoV-2, the cross-immunity was induced against the variants other than the vaccine strain (Wuhan strain).

## Claims

1. A combination vaccine comprising an inactivated whole particle influenza vaccine and an inactivated whole particle SARS-CoV-2 vaccine.

2. The combination vaccine according to claim 1 which does not containing an adjuvant.

3. The combination vaccine according to claim 1 or 2, wherein the combination vaccine is for prevention of influenza infection and COVID-19.

4. The combination vaccine according to any one of claims 1 to 3, wherein the inactivated whole particle influenza vaccine comprises an inactivated whole particle influenza vaccine against two or more influenza virus strains.

5. The combination vaccine according to claim 4, wherein the inactivated whole particle influenza vaccine is a quadrivalent inactivated whole particle influenza vaccine using, as vaccine strains, two type A virus strains and two type B virus strains.

6. The combination vaccine according to any one of claims 1 to 5, wherein the inactivated whole particle influenza vaccine and/or the inactivated whole particle SARS-CoV-2 vaccine is the whole influenza virus and/or the whole SARS-CoV-2 virus which has been inactivated by formaldehyde.

7. An inactivated whole particle SARS-CoV-2 vaccine which is used together with an inactivated whole particle influenza vaccine as a combination vaccine for prevention of influenza infection and COVID-19.

8. An inactivated whole particle influenza vaccine which is used together with an inactivated whole particle SARS-CoV-2 vaccine as a combination vaccine for prevention of influenza infection and COVID-19.

9. A combination of an inactivated whole particle influenza vaccine and an inactivated whole particle SARS-CoV-2 vaccine, wherein the combination is obtained by mixing the inactivated whole particle influenza vaccine and the inactivated whole particle SARS-CoV-2 vaccine at the time of using the combination and is administered to human as a combination vaccine for prevention of influenza infection and COVID-19.

10. The combination according to claim 9, wherein the combination is provided by charging the inactivated whole particle influenza vaccine and the inactivated whole particle SARS-CoV-2 vaccine into different vials.
